# EUROPEAN PATENT APPLICATION

(11) **EP 4 144 343 A1**
(43) Date of publication of application: **08.03.2023**
(21) Application number: 20934169.2
(22) Date of filing: 16.06.2020
(51) Int. Cl.: A61K 8/81, A61K 8/73, A61K 8/46, A61K 8/35, A61K 8/04, A61Q 17/04

(54) **WATER-DISPERSIBLE COSMETIC COMPOSITION FOR BLOCKING ULTRAVIOLET RADIATION COMPRISING DIFFERENT KINDS OF ORGANIC THICKENERS**

(30) Priority: 27.04.2020 KR 20200050986
(71) Applicant: Kolmar Korea Co., Ltd., Sejong 30004 (KR)
(72) Inventor: KIM, Jin Young, Seoul 07255 (KR); BAE, Su Jin, Seoul 05649 (KR); KIM, Jin Mo, Seoul 04188 (KR); HONG, In Ki, Seoul 06261 (KR)
(74) Representative: Gulde & Partner
(86) International application number: PCT/KR2020/007781
(87) International publication number: WO 2021/221228

(57) **Abstract**

The present invention relates to a water-dispersible cosmetic composition for blocking ultraviolet radiation, wherein the composition comprises different kinds of organic thickeners. The water-dispersible cosmetic composition for blocking ultraviolet radiation contains a large amount of moisture, and thus can be frequently reapplied while providing a refreshing sensation and a clean feeling when used.

## Description

### Technical Field

The present invention relates to a sunscreen cosmetic composition in aqueous dispersion form comprising different kinds of organic thickeners, and more particularly, to a sunscreen cosmetic composition in aqueous dispersion form, which gives a cooling feeling and a refreshing feeling of use due to a large amount of water contained therein while being able to be reapplied frequently.

### Background Art

Recently, consumers' awareness of the need for sunscreen in daily life rather than sunscreen during summer or sports and during leisure activities has expanded. Sunscreen products used for this purpose are effective only for a limited period, and thus are recommended to be applied repeatedly every 2 to 3 hours. Therefore, the demand for sunscreen products with a refreshing and light feeling of use has increased.

In general, sunscreen products may be manufactured in various forms such as cream, lotion, stick and powder types. Among them, the creams type that is sold a lot in the market contains a large amount of oil components, and thus has disadvantages in that it leaves a residual oily feeling and feels sticky, which limits frequent reapplication of the cream type. In addition, the cream type contains a large amount of an emulsifying agent, and thus when it comes into contact with water, a white cast phenomenon occurs in which it becomes white and milky while being re-emulsified. In addition, the stick type contains no or tract amount of water, and thus when it is applied to the skin in summer, it is difficult to give a cooling feeling.

Among the types of cosmetics, a gel or skin spray type may be a light and refreshing formulation, but it has a disadvantage in that it is difficult to incorporate a sufficient amount of a sunscreen agent while maintaining the formulation, because the formulation is light in weight. In addition, when a thickener is added to a water-soluble sunscreen agent, dehydration occurs, lowering the viscosity of the formulation. For these reasons, it is very difficult to realize a gel-type formulation which is easy to reapply while being an aqueous dispersion formulation containing a large amount of water.

Therefore, there is a need for the development of a sunscreen cosmetic composition in aqueous dispersion form, which may be continuously re-applied while having a fresh and light feeling of use.

### DISCLOSURE

### Technical Problem

The present invention has been made in order to solve the above-described problems, and an object of the present invention is to provide a sunscreen cosmetic composition in aqueous dispersion form, which may be reapplied frequently while giving a cooling feeling and a refreshing feeling of use due to a large amount of water contained therein.

Objects of the present invention are not limited to the above-mentioned objects, and other objects not mentioned herein will be clearly understood by those skilled in the art from the following description.

### Technical Solution

According to one embodiment of the present invention, there may be provided a sunscreen cosmetic composition comprising a water-soluble sunscreen agent, and different kinds of organic thickeners which consist of a first thickener and a second thickener, the sunscreen cosmetic composition being in aqueous dispersion form.

Preferably, the first thickener may be an acrylic synthetic thickener, and the second thickener may be at least one selected from among polysaccharides and derivatives thereof.

Preferably, the first thickener may include at least one selected from the group consisting of carbomer, an acrylate/C10-30 alkyl acrylate crosspolymer, and mixtures thereof.

Preferably, the second thickener may include at least one selected from the group consisting of hydroxyethyl cellulose, hydroxypropyl cellulose, hydroxypropyl methyl cellulose, hydroxypropyl methyl cellulose stearoxy ether, cellulose gum, xanthan gum, tara gum, sclerotium gum, Acacia Senegal Gum, gellan gum, guar gum, ethyl cellulose, cellulose, carboxymethyl hydroxyethyl cellulose, carboxymethyl cellulose, methyl hydroxyethyl cellulose, hydrolysates thereof, and mixtures thereof.

Preferably, the water-soluble sunscreen agent may include at least one selected from the group consisting of terephthalylidene dicamphor sulfonic acid, disodium phenyl dibenzimidazole tetrasulfonate, phenylbenzimidazole sulfonic acid, benzophenone-4, benzophenone-5, and mixtures thereof.

Preferably, the water-soluble sunscreen agent and the different kinds of organic thickeners may be contained at a ratio of 1: 0.05 to 0.4.

Preferably, the first thickener and the second thickener may be contained at a ratio of 1: 0.02 to 1.5.

Preferably, the first thickener may be contained in an amount of 0.3 to 3.0 wt% based on the total weight of the composition.

Preferably, the water-soluble sunscreen agent may be contained in an amount of 0.5 to 28.0 wt% based on the total weight of the composition.

Preferably, the composition may be in a gel form.

Preferably, the composition may have a viscosity of 35,000 to 150,000 cP.

### Advantageous Effects

The sunscreen cosmetic composition of the present invention is in aqueous dispersion form, is not sticky or greasy due to a large amount of water contained therein, and may give provide a cooling feeling and a refreshing feeling of use.

In addition, even though the sunscreen cosmetic composition of the present invention is in water dispersion form, it may be stably maintained at a viscosity equal to or higher than a certain level while the viscosity is not lowered, due to the use of different kinds of organic thickeners, indicating that the composition may exhibit gel-type properties. Therefore, the composition has advantages in that it has good spreadability and is easy to reapply.

In addition, the sunscreen cosmetic composition of the present invention may have an excellent sunscreen effect due to its high skin adhesion and durability, and may exhibit a significantly reduced degree of a residual oily feeling or a white cast.

### Brief Description of Drawings

In order to more fully understand the drawings referred to in the detailed description of the invention, a brief description of each drawing is presented, in which:

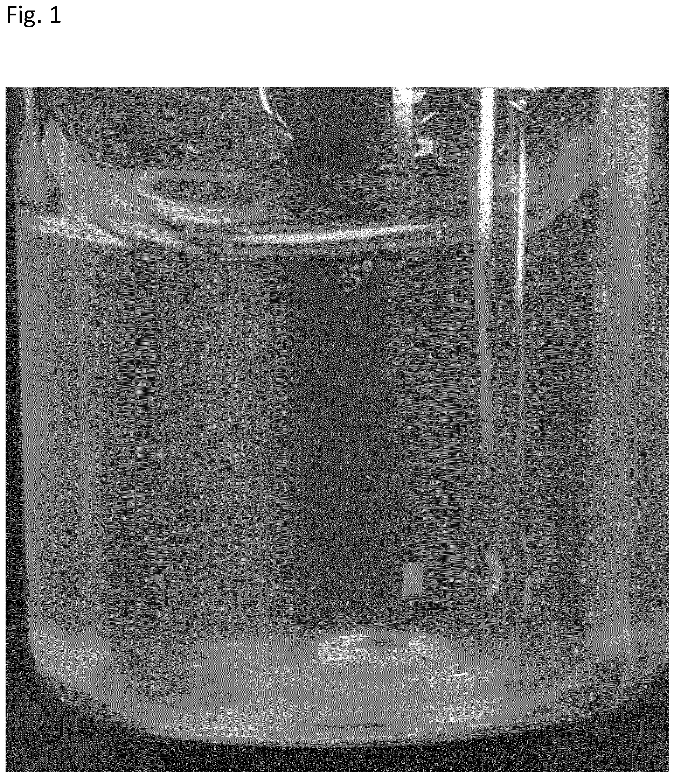

FIG. 1 is a view showing the appearance of a formulation of a composition of Example 1.

### Mode for Invention

Unless otherwise defined, all technical and scientific terms used in the present specification have the same meanings as commonly understood by those skilled in the art to which the present disclosure pertains. In general, the nomenclature used in the present specification is well known and commonly used in the art. In the following description of embodiments of the present invention, the detailed description of related publicly-known configurations or functions will be omitted when it may obscure the understanding of embodiments of the present invention. In addition, embodiments of the present invention will be described below, but the technical spirit of the present invention is not limited or limited thereto, and these embodiments may be modified and variously implemented by those skilled in the art.

The present invention relates to a sunscreen cosmetic composition in aqueous dispersion form, and particularly, to a sunscreen cosmetic composition, the viscosity of which does not decrease and which may be in a stable form, by comprising different kinds of organic thickeners which consist of a first thickener and a second thickener. The sunscreen cosmetic composition in aqueous dispersion form according to the present invention may comprise a water-soluble sunscreen agent, and different kinds of organic thickeners which consist of a first thickener and a second thickener.

Hereinafter, the components of the sunscreen cosmetic composition in aqueous dispersion form according to the present invention will be described in more detail.

In the composition of the present invention, the sunscreen agent is a component having a sunscreen effect, is water-soluble, and may be an organic sunscreen agent. The sunscreen agent may be mixed with and dispersed in a large amount of water due to its water-soluble properties, and when it is an organic sunscreen agent, it has excellent spreadability and causes no white cast.

The water-soluble sunscreen agent of the present invention may include at least one selected from the group consisting of terephthalylidene dicamphor sulfonic acid, disodium phenyl dibenzimidazole tetrasulfonate, phenylbenzimidazole sulfonic acid, benzophenone-4, benzophenone-5, and mixtures thereof.

In the present invention, the water-soluble sunscreen agent may be contained in an amount of 0.5 to 28 wt%, preferably 5 to 15 wt%, based on the total weight of the composition. In one embodiment, the water-soluble sunscreen agent may include 0.5 to 10.0 wt%, preferably 3 to 7 wt%, of terephthalylidene dicamphor sulfonic acid, 0.5 to 10 wt%, preferably 2 to 5 wt%, of disodium phenyl dibenzimidazole tetrasulfonate, and 0.5 to 8 wt%, preferably 2 to 5 wt%, of phenylbenzimidazole sulfonic acid, without being limited thereto.

If the composition of the present invention contains less than 0.5 wt% of the water-soluble sunscreen agent, the sunscreen effect will be insignificant, and if the composition contains more than 28 wt% of the water-soluble sunscreen agent, the formulation stability and feeling of use of the composition may be reduced.

The thickeners of the present invention serve to control the viscosity of the composition, and may include different kinds of organic thickeners which consist of a first thickener and a second thickener. Preferably, the first thickener may be an acrylic synthetic thickener, and the second thickener may be at least one selected from among polysaccharides and derivatives thereof.

The first thickener of the present invention may be a synthetic thickener, specifically an acrylic synthetic thickener. Specifically, the first thickener of the present invention may include at least one selected from the group consisting of carbomer, an acrylate/C10-30 alkyl acrylate crosspolymer, and mixtures thereof.

In the present invention, the first thickener may be contained in an amount of 0.3 to 3.0 wt% based on the total weight of the composition. If the first thickener is contained in an amount of less than 0.3 wt%, a problem may arise in that the viscosity of the cosmetic composition is not maintained at a certain level or higher, making it difficult to maintain the composition in a gel form, and if the first thickener is contained in an amount of more than 3.0 wt%, a problem may arise in that the composition is sticky, and thus it is difficult to apply to the skin and the feeling of use thereof is not good.

It is preferable that the thickeners of the present invention further include a second thickener in addition to the first thickener.

The second thickener that may be mixed with the first thickener may be at least one selected from polysaccharides and derivatives thereof. Specifically, the second thickener of the present invention may be at least one selected from the group consisting of hydroxyethyl cellulose, hydroxypropyl cellulose, hydroxypropyl methyl cellulose, hydroxypropyl methyl cellulose stearoxy ether, cellulose gum, ethyl cellulose, cellulose, carboxymethyl hydroxyethyl cellulose, carboxymethyl cellulose, methyl hydroxyethyl cellulose, xanthan gum, tara gum, sclerotium gum, Acacia Senegal Gum, gellan gum, guar gum, hydrolysates thereof, and mixtures thereof. In addition, the second thickener of the present invention may be added in a form (e.g., hydrolyzed sclerotium gum) made by hydrolyzing the above components with an acid, an enzyme, or other method.

In general, the water-soluble sunscreen agent is an ionic material in which an anion of an acid and a cation of a base are coupled by electrostatic attraction. If the water-soluble sunscreen agent is added in a thickened state formed by dispersing and swelling the thickener in water, a problem arises in that dehydration occurs while the electrostatic attraction is broken, thus reducing the viscosity of the formulation. According to the present invention, it is possible to provide a sunscreen cosmetic composition in stable water dispersion form, the viscosity of which is not reduced even when the water-soluble sunscreen agent is added, by using the above-described kinds of first thickener and second thickener in combination.

In the composition of the present invention, the first thickener and the second thickener may be contained at a ratio of 1: 0.02 to 1.5, preferably 1: 0.07 to 1, most preferably 1: 0.1 to 0.9. When the content ratio between the first thickener and the second thickener is within the above range, the long-term stability of the formulation is high, the transparency of the formulation is high, and the feeling of use is excellent. If the content ratio between the first thickener and the second thickener is less than 1: 0.02, a problem arises in that the feeling of use is reduced, and if the content ratio between the first thickener and the second thickener exceeds 1: 1.5, a problem arises in that the stability and transparency of the formulation are reduced.

In the composition of the present invention, the water-soluble sunscreen agent and the different kinds of organic thickeners may be contained at a ratio of 1: 0.05 to 0.4, preferably 1: 0.08 to 0.25, most preferably 1: 0.1 to 0.2. When the content ratio between the water-soluble sunscreen agent and the thickeners is within the above range, an aqueous dispersion gel formulation may be stably obtained, which has a high sunscreen function and excellent moisturizing ability. If the content ratio between the water-soluble sunscreen agent and the thickeners is less than 1: 0.05, the composition may be in a fluid liquid state due to its low viscosity, the feeling of use thereof may be reduced, and the viscosity thereof may decrease over time. If the content ratio between the water-soluble sunscreen agent and the thickeners is less than 1: 0.4, the thickeners may not be easily dispersed due to high viscosity, the transparency of the composition may be low, the feeling of use of the composition is reduced because the composition is sticky and difficult to apply to the skin, and the formulation stability may be very low.

The water-soluble sunscreen cosmetic composition of the present invention may comprise other ingredients commonly used in the art, for example, at least one selected from among a neutralizing agent, a moisturizing agent, a preservative, a functional additive, a skin conditioning agent, a film-forming agent, a fragrance, a lubricant, an antioxidant, a discoloration inhibitor, a stabilizer, and other additives, within a range that does not impair the effects of the present invention. For example, the composition according to the present invention may comprise tromethamine as a neutralizing agent, butylene glycol as a moisturizing agent, phenoxyethanol as a preservative, and PVP, PVA, etc. as a film-forming agent. However, the present invention is not limited thereto, and the composition of the present invention may further comprise various skin functional additives which are commonly used in the art and components which are comprised in general cosmetic compositions.

The sunscreen cosmetic composition according to the present invention is in aqueous dispersion form. The sunscreen cosmetic composition of the present invention is an aqueous dispersion formulation comprising 70 wt% or more of purified water, and may give a cooling feeling and a refreshing feeling of use due to a large amount of water contained therein.

The sunscreen cosmetic composition according to the present invention may be in a gel form. According to the present invention, it is possible to obtain a gel formulation, the viscosity of which may be stably maintained at a certain level, without lowering the viscosity, by adding the different kinds of organic thickeners to the water-soluble sunscreen agent.

The viscosity of the sunscreen cosmetic composition according to the present invention may be 35,000 cP or more, preferably 35,000 cP to 150,000 cP, more preferably 45,000 cP to 80,000 cP. When the viscosity is within the above range, a stable gel formulation may be obtained.

As such, the present invention has the effect of being able to provide a sunscreen cosmetic composition in water-soluble gel form by adding the different kinds of organic thickeners to the water-soluble sunscreen agent. The sunscreen cosmetic composition in water dispersion form according to the present invention may be stably maintained in a gel form for a long period of time and does not cause a residual oily feeling or a white cast. In addition, the composition of the present invention is easy to reapply frequently, and may give a cooling feeling and a refreshing feeling of use due to a large amount of water contained therein.

Hereinafter, the present invention will be described in more detail with reference to examples, but the present invention is not limited to these examples.

### Preparation Example: Preparation of Examples and Comparative Examples

Sunscreen cosmetic compositions in aqueous dispersion form were prepared using the components shown in Table 1 below. Examples 1 to 5 are sunscreen cosmetic compositions prepared by changing the ratio between different kinds of thickeners, Comparative Example 1 is a sunscreen cosmetic composition to which the second thickener was not added, Comparative Examples 2 to 4 are sunscreen cosmetic compositions prepared by changing the type of second thickener, Comparative Example 5 is a sunscreen cosmetic composition to which the first thickener was not added, Comparative Examples 6 and 7 are sunscreen cosmetic compositions prepared by changing the ratio of the thickeners to the sunscreen agent, and Comparative Example 8 is a sunscreen cosmetic composition prepared by changing the type of the first thickener.

**[Table 1]**

| Role | Component | Ex. 1 | Ex. 2 | Ex. 3 | Ex. 4 | Ex. 5 | Comp . Ex. 1 | Com p. Ex. 2 | Comp. Ex. 3 | Comp. Ex. 4 | Comp . Ex. 5 | Comp . Ex. 6 | Comp . Ex. 7 | Comp . Ex. 8 |
|---|---|---|---|---|---|---|---|---|---|---|---|---|---|---|
| Purified water | Purified water | 72.9 | 72.9 | 73 | 71.9 | 73.4 | 72.0 | 72.9 | 72.9 | 72.9 | 76.4 | 79.1 | 71.4 | 76.4 |
| Thickener | Carbomer | 2.0 | - | 2.0 | 2.95 | 1.15 | 3.2 | 2.0 | 2.0 | 2.0 | - | 0.3 | 3.5 | - |
| | Acrylate/C10-30 alkylacrylate crosspolymer | - | 2.0 | - | - | - | - | - | - | - | - | - | - | - |
| | Hydroxyethyl | - | - | 1.0 | - | - | - | - | - | - | 1.5 | - | - | . |
| | cellulose | | | | | | | | | | | | | |
| | Xanthan gum | 1.0 | 1.0 | - | 0.05 | 1.85 | - | - | - | - | 1.5 | 0.2 | 1.9 | 1.0 |
| | Agar | - | - | - | - | - | - | 1.0 | - | - | - | - | - | - |
| | Sodium polyacrylate | - | - | - | - | - | - | - | 1.0 | - | - | - | - | - |
| | Polyquaternium-37 | - | - | - | - | - | - | - | - | 1.0 | - | - | - | - |
| | PG-150/decyl alcohol/SMDI copolymer | - | - | - | - | - | - | - | - | - | - | - | - | 2.0 |
| | Total weight of thickeners | 3.0 | 3.0 | 3.0 | 3.0 | 3.0 | 3.2 | 3.0 | 3.0 | 3.0 | 3.0 | 0.5 | 5.4 | 3.0 |
| Sunscreen agent | Terephthalylidene dicamphor sulfonic acid | 7.0 | | | | | | | | | | | | |
| | Disodium phenyl dibenzimidazole tetrasulfonate | 3.0 | | | | | | | | | | | | |
| | Phenylbenzimidazol e sulfonic acid | 3.0 | | | | | | | | | | | | |
| Neutralizing agent | Tromethamine | q.s. | q.s. | q.s. | q.s. | q.s. | q.s. | q.s. | q.s. | q.s. | q.s. | q.s. | q.s. | q.s. |
| Moisturizing agent | Butylene glycol | 3.0 | | | | | | | | | | | | |
| Preservative | Phenoxyethanol | 0.4 | | | | | | | | | | | | |

A specific preparation method is as follows.

Phase A was prepared by dispersing the first thickener and other additives such as a moisturizing agent in purified water using an AGI mixer at about 300 to 600 rpm and adjusting the pH to 7.0 or more by the addition of a neutralizing agent. Phase B was prepared by dispersing the second thickener and a suitable amount of a polyol for facilitating dispersion, in purified water using an AGI mixer at 300 to 600 rpm. Then, phase B was added to and dispersed in phase A. Phase C was prepared by dissolving a neutralizing agent in purified water and then adding a sunscreen agent thereto. Then, phase C was added to the mixture of phase A and phase B, mixed uniformly, and then defoamed.

### Experimental Example 1: Formulation and viscosity evaluation

The formulations and transparencies of the compositions according to the Examples and the Comparative Examples were observed, the viscosities thereof measured, and the results are shown in Table 2 below. Viscosity measurement was performed using a Brookfield Viscosmeter LVT with spindle No. 4 and a spindle speed of 6 rpm.

**[Table 2]**

| | Viscosity (cP) | Formulation | Transparency |
|---|---|---|---|
| Example 1 | 70,000 | Gel form | Transparent |
| Example 2 | 55,000 | Gel form | Slightly translucent, but no significant difference when applied to the skin |
| Example 3 | 57,000 | Gel form | Transparent |
| Example 4 | 45,000 | Gel form | Transparent |
| Example 5 | 58,000 | Gel form | Slightly translucent, but no significant difference when applied to the skin |
| Comparative Example 1 | Higher than 150,000 | Dispersion was difficult due to an excessively high content of thickeners | Transparent |
| Comparative Example 2 | Difficult to measure viscosity due to lack of fluidity. | Hard and elastic gel form without fluidity | Suspension |
| Comparative Example 3 | Not measurable | Impossible formulation | - |
| Comparative Example 4 | Not measurable | Impossible formulation | - |
| Comparative Example 5 | 8,000 | Fluid and flowing form | Fine suspension |
| Comparative Example 6 | 12,000 | Fluid and flowing form | Transparent |
| Comparative Example 7 | Higher than 150,000 | Clumped form | Fine suspension |
| Comparative Example 8 | 5,000 | Viscosity continued to increase, resulting in poor viscosity stability | Suspension |

As can be seen in Table 2 above, Examples 1 to 5 had a viscosity of about 45,000 to 80,000 cP and was in a transparent and stable gel form. Representatively, the appearance of the formulation of the composition of Example 1 is shown in FIG. 1. Referring to FIG. 1, it can be confirmed that the transparent contents did not flow and were maintained in a stable gel form.

On the other hand, it can be confirmed that, in the case of Comparative Example 1 to which the second thickener was not added, dispersion was difficult due to high viscosity, and in the case of Comparative Example 5 to which the first thickener was not added, a gel formulation was not obtained due to a very low viscosity, and suspension occurred. Therefore, it can be confirmed that, when the first thickener and the second thickener are used in combination, it is possible to obtain a transparent and stable gel form, unlike when one type of thickener is used.

In addition, in the case of Comparative Example 2 in which "agar" was used as the second thickener, suspension occurred and it was difficult to evenly disperse agar and carbomer in the aqueous phase during preparation. In addition, after the completion of preparation, a hard and elastic gel without fluidity was formed. In the case of Comparative Examples 3 and 4 in which "sodium polyacrylate and polyquaternium-37" were used as the second thickener, a formulation could not be formed, and thus the viscosity could not be measured, and a white gum-like mass was formed immediately after the sunscreen agent was added. In the case of Comparative Example 8 in which the synthetic thickener other than an acrylic thickener was used as the first thickener, problems arose in that suspension occurred, the viscosity was very low, and the viscosity could not be kept constant.

In the case of Comparative Example 6 in which the content ratio between the sunscreen agent and the thickeners was extremely lower than that in the Examples, the composition was in a fluid liquid state due to its very low viscosity, and in the case of Comparative Example 7 in which the content ratio between the sunscreen agent and the thickeners was higher than that in the Examples, a fine suspension occurred, and a flowing down and lumpy appearance appeared due to a very high viscosity.

### Experimental Example 2: Evaluation of feeling of use

In order to evaluate the feeling of use of the compositions according to the Examples and the Comparative Examples, 30 women in their 20s and 40s were asked to evaluate items, including an initial moisturizing feeling, adhesion speed, stickiness after drying, and a soft feeling after drying, and the results are shown in Table 3 below.

In the experimental method, 1 mg/cm² of a sample was taken from the Examples and Comparative Examples and applied to a 3 cm (width) × 2 cm (length) area on the inside of the arm of each subject according to the usual makeup habit, and then each evaluation item was evaluated in 5 stages and scored in units of 1 point according to each stage on a 5-point scale. The feeling of use was evaluated by obtaining the average value of the sum of the scores of 10 people and then excluding the deviation.

### <Evaluation items>

- Initial moisturizing feeling: the higher the moisturizing feeling, the higher the score.
- Adhesion speed: the higher the adhesion speed, the higher the score.
- Stickiness after drying: the lower the stickiness and the higher the freshness, the higher the score.
- Softness after drying: the better the softness, the higher the score.

**[Table 3]**

| | Initial moisturizing feeling | Adhesion speed | Stickiness | Softness |
|---|---|---|---|---|
| Example 1 | 4.9 | 4.8 | 4.9 | 4.5 |
| Example 2 | 4.8 | 4.8 | 4.5 | 4.8 |
| Example 3 | 4.8 | 4.7 | 4.3 | 4.5 |
| Example 4 | 4.7 | 4.7 | 4.3 | 4.0 |
| Example 5 | 4.2 | 4.7 | 4.6 | 4.1 |
| Comparative Example 1 | 3.0 | 4.0 | 3.0 | 1.2 |
| Comparative Example 2 | 3.4 | 1.1 | 3.3 | 3.2 |
| Comparative Example 3 | - | - | - | - |
| Comparative Example 4 | - | - | - | - |
| Comparative Example 5 | 3.2 | 1.3 | 2.1 | 3.2 |
| Comparative Example 6 | 3.8 | 2.4 | 3.6 | 3.7 |
| Comparative Example 7 | 1.2 | 3.7 | 0.2 | 0.5 |
| Comparative Example 8 | 1.8 | 1.2 | 2.1 | 1.1 |
| 1: very bad, 2: bad, 3: moderate, 4: good, 5: very good | | | | |

As can be seen in Table 3 above, the compositions of Examples 1 to 5 obtained high scores in all the items. That is, it can be confirmed that the compositions of Examples 1 to 5 have an excellent moisturizing feeling and good skin adhesion, and may give a soft feeling without being sticky after drying.

On the other hand, it was confirmed that Comparative Example 1, to which the second thickener was not added, did not give a moisturizing feeling due to its stickiness and showed severe skin tightening after drying. Comparative Example 5, to which the first thickener was not added, did not adhere well to the skin and flowed on the skin. Therefore, it can be confirmed that, when the first thickener and the second thickener are used in combination, the feeling of use is excellent compared to when one type of thickener is used.

In addition, Comparative Example 2 comprising "agar" as the second thickener was hard like jelly, and thus could not be applied smoothly onto the skin, and in the case of Comparative Examples 3 and 4 comprising "sodium polyacrylate" or "polyquaternium-37" as the second thickener, the formulation could not be completed and thus the feeling of use could not be tested. It could be confirmed that, in the case of Comparative Example 8 in which a synthetic thickener other than an acrylic thickener was used as the first thickener, the viscosity continued to increase and the viscosity stability was not good.

In the case of Comparative Example 6 in which the content ratio between the sunscreen agent and the thickeners was extremely lower than that in the Examples, and in the case of Comparative Example 7 in which the content ratio between the sunscreen agent and the thickeners was very higher than that in the Examples, the composition could not be evenly applied to the skin due to severe aggregation.

### Experimental Example 3: Evaluation of Long-term stability

Long-term stability evaluation of the compositions according to the Examples and the Comparative Examples was performed, and the results are shown in Table 4 below. Formulation stability was evaluated by placing each composition in a transparent plastic bottle container, maintaining each composition at temperatures of 25°C, 45°C and CYC (-5°C to 40°C), and observing temperature-dependent changes in the viscosity of each composition after one month.

**[Table 4]**

| | Change in viscosity | Change in transparency |
|---|---|---|
| Example 1 | No change | No change |
| Example 2 | No change | No change |
| Example 3 | No change | No change |

| Example 4 | No change | No change |
|---|---|---|
| Example 5 | After about 2 months, the viscosity decreased by about 10% under CYC conditions, and the gel state was maintained. | No change |
| Comparative Example 1 | No change | After a week, suspension occurred under CYC conditions, and precipitation occurred while the part in contact with air was dried |
| Comparative Example 2 | No change | Syneresis and phase separation occurred at all temperatures, and the solubility of the sunscreen agent decreased, causing precipitation. |
| Comparative Example 3 | Not measurable | Not measurable |
| Comparative Example 4 | Not measurable | Not measurable |
| Comparative Example 5 | Viscosity decreased by about more than 30% at all the temperatures | No change |
| Comparative Example 6 | After about 2 weeks, the viscosity decreased under CYC conditions. | No change |
| Comparative Example 7 | Hardened like rubber at all the temperatures. | No change |
| Comparative Example 8 | Viscosity continued to increase at all the temperatures | No change |

As a result, it could be confirmed that, in the compositions of Examples 1 to 5 did not decrease in viscosity or did change in transparency and were maintained in a very stable form for a long period of time.

Meanwhile, in the case of Comparative Example 1, suspension and precipitation occurred, and in the case of Comparative Example 2, syneresis and precipitation occurred. In the case of Comparative Examples 3 and 4, the compositions could not be tested because the formulation was not formed, and in the case of Comparative Examples 5 to 7, the viscosity decreased, or the composition was hardened like rubber at all the temperatures. In the case of Comparative Example 8, a phenomenon occurred in which the viscosity continued to increase at all the temperatures. Accordingly, it could be confirmed that, in the case of the compositions of Comparative Examples, the formulation was not stably maintained for a long time while the viscosity or transparency greatly changed.

### Experimental Example 4: Measurement of sun protection factor (SPF)

The sun protection factor (SPF) values of the cosmetic compositions according to Examples 1 to 5 and Comparative Examples 1, 2 and 5 to 8 were evaluated. Specifically, in order to evaluate the sun protection factor (SPF) values, 1.3 mg/cm² of a sample was taken from each composition and applied to a polymethyl methacrylate (PMMA; HELIO PLATE HD6) plate for about 30 seconds, and the applied sample was dried in the dark for 15 minutes. The SPF value of each dried sample was measured using the Labsphere UV 2000S *in vitro* SPF analyzer. The sun protection factor value was expressed as the average value of three experimental values, and the results are shown in Table 5 below.

**[Table 5]**

| | SPF value | | SPF value |
|---|---|---|---|
| Example 1 | 53 | Comparative Example 1 | 15 |
| Example 2 | 44 | Comparative Example 2 | 12 |
| Example 3 | 50 | Comparative Example 3 | - |
| Example 4 | 35 | Comparative Example 4 | - |
| Example 5 | 48 | Comparative Example 5 | 12 |
| | | Comparative Example 6 | 5 |
| | | Comparative Example 7 | 18 |
| | | Comparative Example 8 | 10 |

As a result, it could be confirmed that the compositions of Examples 1 to 5 had a high sun protection factor (SPF) value of 30 or higher. On the other hand, it could be confirmed that the compositions of the Comparative Examples had a significantly low sun protection factor (SPF) value because the function of the sunscreen agent could not be maintained.

As can be seen from the above results, the sunscreen cosmetic composition in water dispersion form according to the present invention has a high moisturizing feeling, a fresh and excellent feeling of use, and high UV protection ability, and may provide a stable gel formulation by containing different kinds of organic thickeners.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A sunscreen cosmetic composition comprising a water-soluble sunscreen agent, and different kinds of organic thickeners which consist of a first thickener and a second thickener, wherein the sunscreen cosmetic composition is in aqueous dispersion form.

2. The sunscreen cosmetic composition according to claim 1, wherein the first thickener is an acrylic synthetic thickener, and the second thickener is at least one selected from among polysaccharides and derivatives thereof.

3. The sunscreen cosmetic composition according to claim 1, wherein the first thickener comprises at least one selected from the group consisting of carbomer, an acrylate/C10-30 alkyl acrylate crosspolymer, and mixtures thereof.

4. The sunscreen cosmetic composition according to claim 1, wherein the second thickener comprises at least one selected from the group consisting of hydroxyethyl cellulose, hydroxypropyl cellulose, hydroxypropyl methyl cellulose, hydroxypropyl methyl cellulose stearoxy ether, cellulose gum, xanthan gum, tara gum, sclerotium gum, Acacia Senegal Gum, gellan gum, guar gum, ethyl cellulose, cellulose, carboxymethyl hydroxyethyl cellulose, carboxymethyl cellulose, methyl hydroxyethyl cellulose, hydrolysates thereof, and mixtures thereof.

5. The sunscreen cosmetic composition according to claim 1, wherein the water-soluble sunscreen agent comprises at least one selected from the group consisting of terephthalylidene dicamphor sulfonic acid, disodium phenyl dibenzimidazole tetrasulfonate, phenylbenzimidazole sulfonic acid, benzophenone-4, benzophenone-5, and mixtures thereof.

6. The sunscreen cosmetic composition according to claim 1, wherein the water-soluble sunscreen agent and the different kinds of organic thickeners are contained at a ratio of 1: 0.05 to 0.4.

7. The sunscreen cosmetic composition according to claim 1, wherein the first thickener and the second thickener are contained at a ratio of 1: 0.02 to 1.5.

8. The sunscreen cosmetic composition according to claim 1, wherein the first thickener is contained in an amount of 0.3 to 3.0 wt% based on the total weight of the composition.

9. The sunscreen cosmetic composition according to claim 1, wherein the water-soluble sunscreen agent is contained in an amount of 0.5 to 28.0 wt% based on the total weight of the composition.

10. The sunscreen cosmetic composition according to claim 1, wherein the composition is in a gel form.

11. The sunscreen cosmetic composition according to claim 1, wherein the composition has a viscosity of 35,000 to 150,000 cP.
